# EUROPEAN PATENT SPECIFICATION

(11) **EP 0 628 293 B1**
(45) Date of publication and mention of the grant of the patent: **28.11.2001**
(21) Application number: 94201567.8
(22) Date of filing: 02.06.1994
(51) Int. Cl.: A61F 2/30

(54) **Temporomandibular joint prosthesis**
Kiefergelenkprothese
Prothése de l'articulation temporomandibulaire

(30) Priority: 02.06.1993 EP 93201583
(43) Date of publication of application: 14.12.1994
(73) Proprietor: ACADEMISCH ZIEKENHUIS GRONINGEN, NL-9713 EZ Groningen (NL)
(72) Inventor: Falkenström, Che Hsin, NL-7511 HX Enschede (NL)
(74) Representative: Smulders, Theodorus A.H.J., Ir.

(56) References cited:
- EP-A- 0 203 719
- WO-A-87/04917
- FR-A- 2 558 721

## Description

The invention relates to a temporomandibular joint prosthesis comprising a cranial prosthesis-part comprising a support surface adapted to be seated against bone tissue in the area of and/or adjacent to the natural glenoid fossa and/or the articular eminence and a mandibular prosthesis-part adapted to be pivotable relative to the cranial prosthesis-part when in implanted condition.

A treatment to counteract dysfunction of natural joints by causes such as ankylosis, osteoarthrosis, tumour or developmental disorders is to partially or completely replace the natural joint by a prosthesis.

For the treatment of dysfunctions of the temporomandibular joint, maxillofacial surgeons generally prefer a total temporomandibular joint prosthesis as identified in the preamble to preclude any risk of intrusion of the artificial condylar head into the midcranial fossa through the fossa roof or the possibility of condylar head resorption.

The natural temporomandibular joint allows both rotation and essentially horizontal translation of the mandibular condyle. Translation is controlled by lateral pterygoid muscles which are attached to the mandibular condyle. The function of these muscles is lost when the mandibular condyle to which they are attached is replaced by a mandibular prosthesis-part.

Examples of such a total temporomandibular joint prostheses are described in: 'Total Prosthetic Replacement of Temporomandibular Joint' by C.D. Kiehn et al. in Ann Plast Surg, 1979; 2:5 and in: 'Temporomandibular Joint Condylar Prosthesis: A Ten Year Report', by J.N. Kent et al. in Journal of Oral Maxillofacial Surgery, 1983; 41:245-254. In these and other known prostheses of this type, the anatomical shape of the natural articulating surfaces, i.e. the mandibular condyle, the articular eminence and the glenoid fossa, is essentially copied.

After implantation of a known prosthesis as discussed above, the mandible can only rotate about a fixed point at the side of the prosthesis, which point essentially corresponds with the centre of the replaced natural mandibular condyle, so instead of the muscular control of the translatory movement of the jaw a fixed point of rotation is obtained, precluding translatory movement of the mandible (lower jaw) at the side where the prosthesis is implanted.

This is uncomfortable for the patient and impairs the function of the mandible. Furthermore, rotation of the jaw about a substantially vertical axis is caused when a translation occurs in the opposite joint. This unnatural rotation causes exertion of abnormal loads on the opposite joint and thus an increased risk of dysfunction of the opposite natural joint. This is particularly undesirable when the prosthesis is implanted in a patient suffering from a disease which tends to affect the joints in general. Because of the above-described disadvantages of known temporomandibular joint prostheses, temporomandibular joint replacements are seldom performed in practice.

It is an object of the present invention to overcome these disadvantages by providing a temporomandibular joint prosthesis which provides a better user comfort and an improved function of the mandible, and allows a more natural mandibular motion.

According to the present invention, this object is achieved by providing a temporomandibular joint prosthesis of the above-described type in which the mandibular prosthesis-part is pivotable about a centre of rotation which is located in such a position relative to the support surface of the cranial prosthesis-part, that in implanted condition the centre of rotation is located inferior to the centre of the natural mandibular condyle.

Due to these features, a more natural motion of the mandible, including translational movement of the prosthesis-portion located at the position of the replaced mandibular condyle, is obtained in spite of the loss of the function of the lateral pterygoid muscles.

The invention is based on the insight that to achieve the above-mentioned objects, the designer should primarily aim at restoration of the biomechanical functions of the joint, whereas hitherto designers were focused predominantly on providing an anatomical structure looking similar to the natural structure to be replaced.

The invention can also be embodied in a cranial prosthesis-part for use as part of a prosthesis according to a particular embodiment of the invention having a fixed centre of rotation of the mandibular prosthesis-part. This cranial prosthesis-part comprises a support surface adapted to be seated against bone tissue in the area of or adjacent to the natural glenoid fossa and/or the articular eminence, a sphere-shaped cavity or convex element, the centre defined by said sphere being located in such a substantially fixed position relative to the support surface, that in implanted condition said centre is located between 5 and 25 mm, and preferably about 15 mm, inferior to the centre of the natural mandibular condyle.

Further particular embodiments of the invention are set out in the claims 2-14 and 16.

Hereinafter, the invention will be further explained and elucidated on the basis of four embodiments of the present invention, with reference to the drawings, in which:
Fig. 1 is a side view in cross-section along the line B-B in Fig. 2 of an implanted right-side prosthesis according to the invention,
Fig. 2 is a side view in cross-section along the line A-A in Fig. 1,
Figs. 3-5 are schematical side views of second, third and fourth implanted prostheses according to the invention, and
Fig. 6 is a side view in cross-section of yet another implanted right-side prosthesis according to the invention.

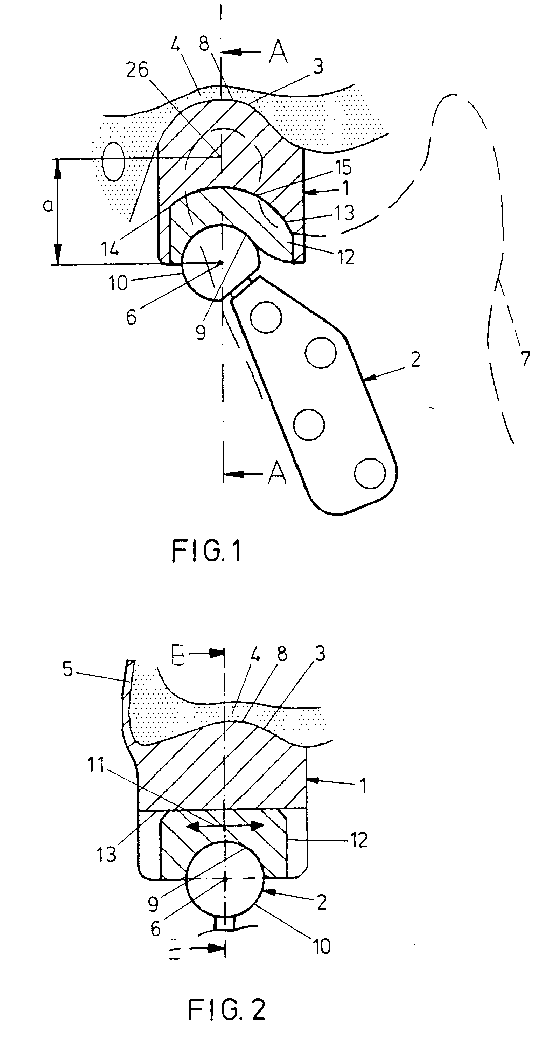

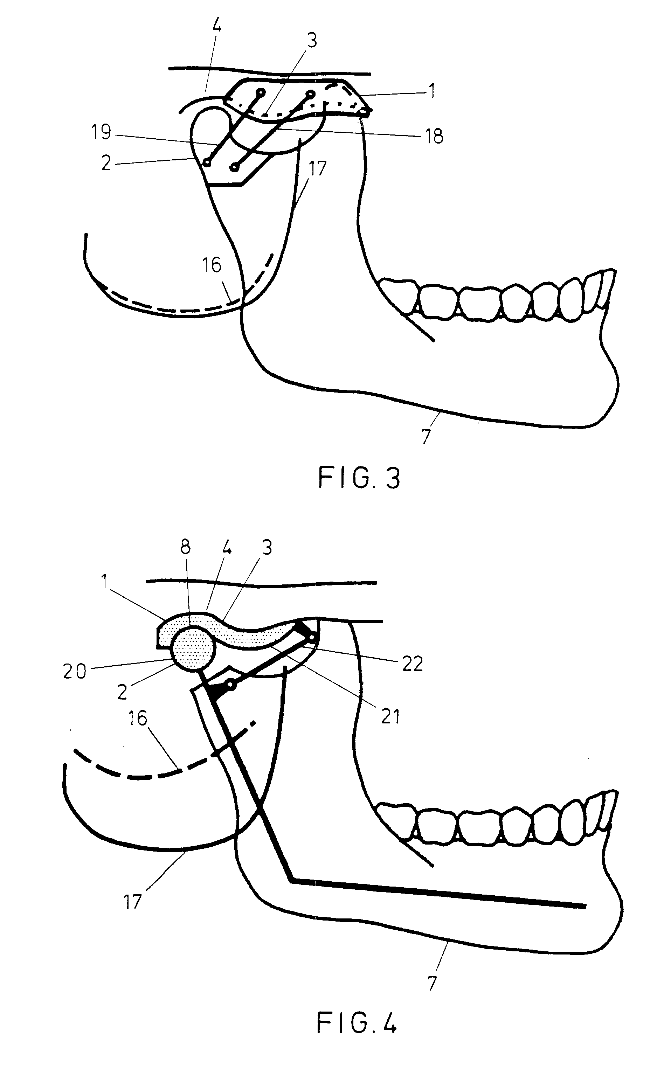

The invention is first described with reference to the embodiment shown in Figs. 1 and 2. Subsequently the other embodiments shown in Figs. 3-5 will be described and elucidated. Corresponding parts of the various embodiments are designated by identical reference numerals.

In Figs. 1 and 2 a temporomandibular joint prosthesis comprising a cranial prosthesis-part 1 and a mandibular prosthesis-part 2 is shown.

The cranial prosthesis-part may be provided with a support surface adapted and located to be fitted against a lateral side 5 of the zygomatic arch and/or the articular tubercle as is usual with known prostheses. The fossa prosthesis-part will then be supported mainly by bone screws anchored in the zygomatic arch and/or the articular tubercle. It is however preferred, to provide the cranial prosthesis-part 1, instead or in addition, with a support surface 3 adapted and located to be fitted to bone tissue 4 of the natural glenoid fossa and/or the articular eminence, so that an improved load distribution and support of the fossa prosthesis-part 1 is obtained. The support surface 3 shown in Figs. 1 and 2 is fitted to both the glenoid fossa and a part of the articular eminence to obtain an optimal load distribution. The support surface 3 may be fitted to a large degree prior to implantation if the shape of the fossa is determined using for example computer tomography. The shape of the support surface 3 of a standard or customized prosthesis may be fitted or more accurately fitted using bone cement such as PMMA, which is however less bio-inert than suitable materials for manufacturing the cranial prosthesis-part.

The mandibular prosthesis-part 2 is adapted to be pivotable relative to the cranial prosthesis-part 1 about a centre of rotation 6 which is located in such a position relative to the support surface 3 of the cranial prosthesis-part 1, that in implanted condition the centre of rotation 6 is located inferior to the centre 26 of the natural mandibular condyle.

This allows a natural motion of the mandible 7 including translational movement of the prosthesis 2 replacing the mandibular condyle in spite of the loss of the function of the lateral pterygoid muscles.

To achieve that in implanted condition the centre of rotation is located inferior to the centre 26 of the natural mandibular condyle, in a prosthesis of which the support surface 3 of the cranial prosthesis-part 1 is adapted to be fitted to bone tissue 4 of the fossa, the centre of rotation 6 will be located in a plane parallel to and at a distance of more than 11 mm from a central portion 8 of that part of the support surface 3 of the cranial prosthesis-part 1. After implantation, this central portion 8 will be fitted to a central portion of the fossa. In addition, the support surface 3 may also be supported by other bone tissue, as is shown in the drawings.

The required distance between the central portion 8 of the support surface 3 and the plane to obtain a predetermined distance between the centre of the former natural mandibular condyle and the centre of rotation 6 may vary somewhat dependent from the morphology of the patient (in particular the depth of the fossa) and from the intended use of bone cement between the fossa prosthesis-part 1 and the bone tissue 4 of the fossa.

The most natural motion of the mandible 7 is obtained if the centre of rotation 6 is located in such a position relative to the support surface 3 of the cranial prosthesis-part 1, that in implanted condition that centre of rotation 6 is located between 5 and 60 mm inferior to the centre of the natural mandibular condyle .

To achieve this amount of inferior displacement of the centre of rotation 6 relative to the centre 26 of the natural mandibular condyle in a prosthesis of which the support surface 3 of the cranial prosthesis-part 1 is adapted to be fitted to bone tissue 4 of the fossa, the centre of rotation 6 will be located in a plane parallel to and at a distance of between 13 and 68 mm from a central portion 8 of the support surface 3 of the cranial prosthesis-part 1.

In the prosthesis shown in Figs. 1 and 2, the mandibular prosthesis-part 2 is, at least in side-view, pivotable about a single centre of rotation 6. This is advantageous for obtaining a simple reliable construction with a long lifetime, in particular regarding wear, and requiring little space. For these reasons, presently a construction with a single centre of rotation is preferred over constructions in which an instantaneous centre of rotation moves along a path (centrode) when the mandible is articulated, although with the latter type of constructions (see Figs. 3-5) a somewhat better reproduction of the motion of the mandible supported by a healthy, natural joint can be achieved.

For prostheses with a single centre of rotation 6 for obtaining an optimal fit of the motion of the mandible after implantation relative to the mandible articulated by a natural, healthy joint, the centre of rotation should preferably be located in such a position relative to the support surface 3 of the cranial prosthesis-part 1, that in implanted condition that centre of rotation 6 is located between 5 and 25 mm inferior to the centre of the natural mandibular condyle.

Best results regarding avoidance of rotation of the intercondylar axis in frontal and transversal planes, avoidance of mediolateral movement of the contralateral condyle and avoidance of overstretching of the temporomandibular ligament at the side of the prosthesis are generally achieved with single centre of rotation prostheses if the centre of rotation 6 is located 15 mm inferior to the centre of the natural mandibular condyle.

In combination with this inferior displacement, an anterior displacement of the centre of rotation 6 relative to the centre of the natural mandibular condyle of up to 5 mm and also a slight posterior displacement provides good results from a kinematic point of view.

To obtain the above-mentioned inferior displacements of 5 to 25 mm and preferably 15 mm in a prosthesis of which the support surface 3 is adapted to be fitted to bone tissue 4 of the fossa, the centre of rotation 6 is to be located in a plane parallel to and at a distance between 13 and 33 mm, respectively of about 23 mm, from a central portion 8 of the support surface 3 of the cranial prosthesis-part 1.

The cranial prosthesis-part 1 shown in Figs. 1 and 2 comprises a sphere-shaped cavity 9 and the mandibular prosthesis-part 2 comprises a sphere-shaped convex element 10 adapted to be seated in the cavity 9 with a sliding fit. Thus the forces exerted on the joint are always well distributed over the bearing surfaces. This is of particular importance in a temporomandibular joint prosthesis, because of the important forces exerted thereon and the influence of surface pressure on wear of biologically suitable bearing materials, which was and still is a major concern in the field of artificial joint design. The radial clearance between the convex element 10 and the cavity 9 is preferably about 0.1 mm but dependent on the elasticity of the bearing material other amounts of clearance may be preferable.

It is noted, that in principle the convex element may also be provided on the cranial prosthesis-part and the concavity may also be provided on the mandibular prosthesis-part.

A problem which occurs when implanting a temporomandibular prosthesis is, that the prosthesis-parts have to be exactly positioned relative to each other to avoid a pretension in both the contralateral joint and the implanted, artificial joint, i.e. the distance between the skull-side and the mandible side bearing-surfaces of the joints should correspond exactly. Due to such pretensions, additional forces are exerted on the temporomandibular joints causing additional wear and friction.

In the prosthesis shown in Figs. 1 and 2, this problem is solved by adapting the prosthesis-parts 1 and 2 for translational displacement relative to each other in directions 11 which are oriented essentially medial and lateral when the prosthesis is in implanted condition. The exact direction of the allowed relative movements in implanted condition may be exactly lateral and medial, but also may also be somewhat oblique, for example at an angle of 10 - 20° to the transversal plane.

Thus the prosthesis-parts are not mutually fixed in medial-lateral direction 11 and can adapt to their relative medial-lateral positions. Moreover, medial and lateral movement of the mandible is allowed, for example during chewing.

Since in the prosthesis shown in Figs. 1 and 2 the centre of rotation 6 is displaced away from the glenoid fossa relative to the position of the centre of the natural mandibular condyle, sufficient room is present between the fossa and the sphere-shaped convex element 10 of the mandible condyle prosthesis-part 2 for a traveller 12 provided with the sphere-shaped cavity 9 or the sphere-shaped convex element 10 and a guide 13. The traveller 12 is movable along a guide 13 in the direction 11 of translational displacement. Thus a simple and reliable means of providing the desired medial and lateral translational movability is obtained.

The guide 13 is provided in form of a channel 12 having a composite cross-section with, in implanted condition, a posterior edge and an anteriorly and upwardly facing rounded portion 15. The traveller is shaped correspondingly. Due to this shape, surface pressure is kept relatively low, by providing a maximum surface extending perpendicular to the major forces exerted on the joint.

The traveller 12 can for example be made from a suitable polymer such as UHMWPE. For the condyle for example Alumina (Al₂O₃) would be suitable. For the basis of the cranial prosthesis-part 1 an Co-Cr-Mo alloy is preferred to keep wear low, although this material is less bio-inert than suitable titanium alloys. To alleviate this problem, the support surface 3 of the cranial prosthesis-part 1 may be covered with hydroxylapatite, which stimulates bone ingrowth. Screws and the stem of the mandibular prosthesis-part 2 are preferably made out of a titanium alloy.

The screws are preferably fixed in titanium implants with thread inside to avoid damage of the bone when the screws are screwed out in the event the prosthesis has to be removed for replacement or reconditioning. Such screw/implant combinations can also advantageously be used for anchoring other types of implants which sometimes have to be replaced or reconditioned.

In Figs. 3-5 three embodiments of the invention in which the mandibular prosthesis-part 2 is movable relative to the cranial prosthesis-part 1 such that upon rotation of the mandibular prosthesis-part 2, an instantaneous centre of rotation of the mandible moves along a centrode 16. This allows a better fit of the movement of the mandible 7 to the movement of the mandible supported by a natural temporomandibular joint. Research by the applicant has revealed that the centrode of the mandible 7 supported by a natural temporomandibular joint is generally U-shaped, the open side of the path facing upwards, generally as the paths 17 shown in Figs. 3-5.

The best fit of the mandibular motion with respect to the natural mandibular motion is thus obtained by a prosthesis of which the centrode 16 is U-shaped, the open end of the U-shaped centrode facing towards the cranial prosthesis-part. The centrode 16 of the prosthesis shown in Fig. 3 and, to a lesser extent, the centrode 16 of the prosthesis shown in Fig. 4 have such a shape. Not only the centrodes of movement of the mandibular parts of these prostheses fit very well to the natural corresponding centrodes, also the articulation/translation curves of the mandibular condyles replacing parts fit very well to the natural curves.

As appears from the Fig. 3, a very close fit of the natural and the prosthesis centrodes 17 resp. 16 is obtained if the prosthesis-parts 1 and 2 are connected by two bars 18, 19 hinged between the prosthesis-parts 1 and 2, such that the prosthesis-parts 1, 2 and the bars 18, 19 form a four bar system. If sufficient play is provided in the hinges, the prosthesis also provides the possibility of essentially medial and lateral translational displacement of the prosthesis-parts 1 and 2 relative to each other.

The prosthesis shown in Fig. 4 has a mandibular prosthesis-part 2 comprising an element 20 with a convex bearing surface and a cranial prosthesis-part 1 provided with a bearing surface 21 extending mainly in one direction. The bearing surface 21 is disposed such that in implanted condition the element 20 can slide ventrally and dorsally along the bearing surface 21 of the cranial prosthesis-part 1. Furthermore, an artificial ligament 22 is attached to the element 20 in a position spaced from its bearing surface. Thus a more simple and reliable design, with nevertheless a substantially more natural motion of the mandible 7 is obtained.

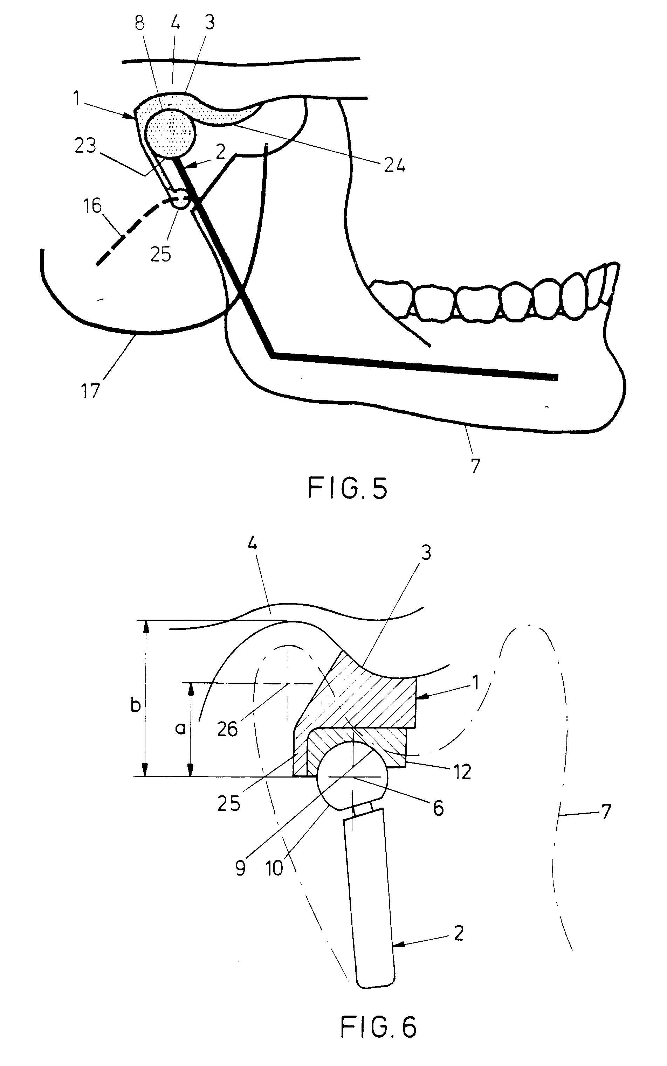

The prosthesis shown in Fig. 5 is of a further simplified design, but still allows a substantially more natural movement of the mandible 7 than known temporomandibular prostheses. This is achieved by providing the mandibular prosthesis-part 2 with an element 23 with a convex bearing surface, providing the cranial prosthesis-part 1 with a bearing surface 24 extending mainly in one direction and providing a constraint 25 spaced from and in front of its bearing surface 24 for posteriorly supporting an adjacent part of the mandibular prosthesis-part 2. The bearing surfaces 24 are disposed such that in implanted condition the element 23 can slide ventrally and dorsally along the bearing surface 24 of the cranial prosthesis-part 1.

Like the prosthesis shown in Figs. 1 and 2, the prosthesis shown in Fig. 6 comprises cooperating bearing surfaces 9, 10 which fit to each other with a sliding fit and are curved about the centre of rotation 6, which forms a centre of curvature of these bearing surfaces. In implanted condition, as shown, the centre of rotation of the cooperating bearing surfaces 9, 10 is located spaced below the position 26 of the centre of the replaced mandibular condyle when at rest in the glenoid fossa.

The traveller 12 carrying one of the cooperating bearing surfaces is movable in forward and backward direction, similar to the convex element 8 of the prosthesis shown in Fig. 5 and also in lateral direction similar to the traveller 12 of the prosthesis shown in Figs. 1 and 2. The horizontal freedom of movement of the traveller 12 allows the jaw to move freely along natural patterns of motion. In particular, it leaves the jaw free to move with a forward and a backward component during chewing.

Since the centre of rotation 6 of the bearing means 10, 12 is positioned spaced below the position 26 of the centre of the replaced natural mandibular condyle, the mandibular prosthesis part 2 is in effect hinged to the cranial prosthesis part 1 to pivot about a centre of rotation 6 which is located lower than in known prostheses in which the centre of rotation of the hinge between the cranial prosthesis part and the mandibular prosthesis part is located essentially at the same horizontal level as the centre of the replaced natural mandibular condyle or as the centre defined by the radius of the glenoid fossa.

The lowered position of centre of rotation 6 of the hinge means, relative to the centre 26 of the healthy natural mandibular condyle when at rest in the glenoid fossa allows a more natural movement of the jaw, and in particular of the mandibular ascending ramus to which the mandibular prostheis-part is fixed, without requiring any translational displacement of prosthesis parts adjacent to the glenoid fossa and the articular eminence. Translational movements of prosthesis parts adjacent the glenoid fossa and the articular eminence are generally rendered impossible or at least substantially inhibited by scar tissue the patient develops in that area. It is for this reason, that known prostheses which theoretically allow the condylar head to translate along a cover of the glenoid fossa and the articular eminence (as known from EP-A-0 203 719) often do not allow the natural movement which would be expected.

Moreover, room beneath the zygomatic arch for the traveller 12 is obtained and the artificial mandibular condyle can be positioned forwardly relative to the position 26 of the centre of the healthy, natural mandibular condyle when at rest in the glenoid fossa.

A forward position of the centre of rotation 6 of the hinge between the cranial prosthesis part 1 and the mandibular prosthesis part 2 is advantageous, because loads exerted via the mandibular prosthesis part 2 onto the cranial prosthesis part 1 are introduced more forwardly, which allows to seat the support surface 3 of the cranial prosthesis part 1 accordingly further forwardly, i.e. essentially against the articular eminence which in a natural joint supports the condylar head when one tensions the jaw muscles during biting and chewing. Thus, loads can be passed to a cranial bony area which is by nature suitable for resisting such loads.

As was discussed earlier, the distance a between the position 26 of the centre of the natural mandibular condyle in the glenoid fossa and the centre of rotation 6 of the hinge between the cranial prosthesis part 1 and the mandibular prosthesis part 2 is preferably at least 5 mm. Similarly to what was described in connection with embodiments shown in Figs. 1, 2, 4 and 5, which comprise a support surface adapted to be seated against the glenoid fossa, this brings about that the centre of rotation 6 will typically be in a plane parallel to a central surface portion of the glenoid fossa and at a distance b of at least 13 mm from that central surface portion of the glenoid fossa.

Similar to the prosthesis shown in Fig. 5, the prosthesis shown in Fig. 6 is provided with a constraint 25, which provides posterior support for the mandibular prosthesis part 2. In the embodiment shown in Fig. 6, this is achieved since the constraint 25 precludes posterior movement of the traveller to which the mandibular prosthesis part 2 is hinged beyond a predetermined position. The function of this constraint 25 is primarily to avoid dislocation of the traveller 12 when unusual backwardly directed loads are exerted onto the jaw. However, it may also provide some support to compensate for the loss of the action of the lateral pterygoid muscles.

## Claims

1. A temporomandibular joint prosthesis comprising a cranial prosthesis-part (1) comprising a support surface (3) adapted to be seated against bone tissue (4) in the area of and/or adjacent to the natural glenoid fossa and/or the articular eminence and a mandibular prosthesis-part (2) adapted to be pivotable relative to the cranial prosthesis-part (1) when in implanted condition, **characterized in that**, the mandibular prosthesis-part (2) is pivotable about a centre of rotation (6, 16) which is located in such a position relative to the support surface (3) of the cranial prosthesis-part (1), that in implanted condition said centre of rotation (6) is located inferior to the centre (26) of the natural mandibular condyle.

2. A prosthesis according to claim 1, wherein the support surface (3) is adapted to be fitted to bone tissue (4) of the fossa and the centre of rotation (6, 16) is located in a plane parallel to and at a distance of more than 11 mm from a central portion (8) of said support surface (3) of the cranial prosthesis-part (1).

3. A prosthesis according to claim 1 or 2, wherein said centre of rotation (6, 16) is located in such a position relative to the support surface (3) of the cranial prosthesis-part (1), that in implanted condition said centre of rotation (6, 16) is located between 5 and 60 mm inferior to the centre (26) of the natural mandibular condyle.

4. A prosthesis according to one of the preceding claims, wherein the support surface (3) is adapted to be fitted to bone tissue (4) of the fossa and the centre of rotation (6, 16) is located in a plane parallel to and at a distance between 13 and 68 mm from a central portion (8) of the support surface (3) of the cranial prosthesis-part (1).

5. A prosthesis according to one of the preceding claims, wherein the mandibular prosthesis-part (2) is pivotable about a single centre of rotation (6) which is located in such a substantially fixed position relative to the support surface (3) of the cranial prosthesis-part (1), that in implanted condition said centre of rotation (6) is located between 5 and 25 mm, and preferably about 15 mm, inferior to the centre (26) of the natural mandibular condyle.

6. A prosthesis according to one of the preceding claims, wherein the mandibular prosthesis-part (2) is pivotable about a single centre of rotation (6) which is located in a substantially fixed position relative to the support surface (3) of the cranial prosthesis-part (1), the support surface (3) is adapted to be fitted to bone tissue (4) of the fossa and the centre of rotation (6, 16) is located in a plane parallel to and at a distance between 13 and 33 mm, and preferably about 23 mm, from a central portion (8) of the support surface (3) of the cranial prosthesis-part (1).

7. A prosthesis according to one of the preceding claims, wherein one of said prosthesis-parts (1, 2) comprises a sphere-shaped cavity (9) and the other one of said prosthesis-parts comprises a sphere-shaped convex element (10) adapted to be seated in said cavity with a sliding fit.

8. A prosthesis according to one of the claims 1-4, wherein the mandibular prosthesis-part (2) is movable relative to the cranial prosthesis-part (1) such that upon rotation of the mandibular prosthesis-part (2), an instantaneous centre of rotation of the mandible moves along a centrode (16).

9. A prosthesis according to claim 8, wherein the centrode (16) is U-shaped, the open end of the U-shaped centrode (16) facing towards the cranial prosthesis-part (1).

10. A prosthesis according to claim 9, wherein two bars (18, 19) are hinged between said prosthesis-parts (1, 2), the prosthesis-parts (1, 2) and the bars (18, 19) forming a four bar system.

11. A prosthesis according to claim 8, wherein the mandibular prosthesis-part (2) comprises an element (20) with a convex bearing surface, the cranial prosthesis-part (1) is provided with a bearing surface (21) extending mainly in one direction, the bearing surfaces (21) being disposed such that in implanted condition the element (20) can slide ventrally and dorsally along the bearing surface (21) of the cranial prosthesis-part (1), and an artificial ligament (22) is attached to the element in a position spaced from its bearing surface (21).

12. A prosthesis according to claim 8, wherein the mandibular prosthesis-part (2) comprises an element (23) with a convex bearing surface, the cranial prosthesis-part (1) is provided with a bearing surface (24) extending mainly in one direction and a constraint (25) spaced from and in front of its bearing surface (24) for posteriorly supporting an adjacent part of the mandibular prosthesis-part (2), the bearing surface (24) being disposed such that in implanted condition the element (23) can slide ventrally and dorsally along the bearing surface (24) of the cranial prosthesis-part (1)

13. A prosthesis according to one of the preceding claims, wherein the prosthesis-parts (1, 2) are adapted for translational displacement relative to each other in directions (11) which are essentially medially and laterally oriented when the prosthesis is in implanted condition.

14. A prosthesis according to claim 13, wherein one of said prosthesis-parts (1, 2) comprises a sphere-shaped cavity (9) and the other one of said prosthesis-parts comprises a sphere-shaped convex element (10) adapted to be seated in said cavity (9) with a sliding fit, and the glenoid fossa prosthesis part (1) comprises a traveller (12) movable along a guide (13) in said direction (11) of translational displacement and provided with the sphere-shaped cavity (9) or the sphere-shaped convex element (10).

15. A glenoid fossa prosthesis for use as part of a prosthesis according to claim 7 comprising a support surface (3) adapted to be seated against bone tissue (4) in the area of or adjacent to the natural glenoid fossa and/or the articular eminence, a sphere-shaped cavity (9) or convex element (10), the centre (6) defined by said sphere-shaped cavity (9) or convex element (10) being located in such a substantially fixed position relative to the support surface (3), that in implanted condition said centre is located between 5 and 25 mm, and preferably about 15 mm, inferior to the centre (26) of the natural mandibular condyle.

16. A glenoid fossa prosthesis according to claim 15, wherein said support surface (3) is adapted to be fitted to bone tissue (4) of the natural glenoid fossa, and wherein the centre (6) defined by said sphere-shaped cavity (9) or convex element (10) is located in a plane parallel to and at a distance between 13 and 33 mm, and preferably of about 23 mm, from a central portion of the support surface (3).

## Patentansprüche

1. Kiefergelenkprothese mit einem schädelseitigen Prothesentell (1) mit einer Stützfläche (3), die im Bereich der und/oder nahe der natürlichen Gelenkpfanne und/oder des Gelenkvorsprungs an Knochengewebe (4) gesetzt werden kann, und einem kieferseitigen Prothesenteil (2), das im implantierten Zustand relativ zum schädelseitigen Prothesenteil (1) verschwenkbar ist, **dadurch gekennzeichnet, daß** das kieferseitige Prothesenteil (2) um einen Drehmittelpunkt (6, 16) drehbar ist, der derart in bezug zur Stützfläche (3) des schädelseitigen Prothesenteil (1) positioniert ist, daß, im implantierten Zustand, der Drehmittelpunkt (6) unterhalb der Mitte (26) des natürlichen Klefergelenkkopfs liegt.

2. Prothese nach Anspruch 1, bei der die Stützfläche (3) an Knochengewebe (4) der Gelenkpfanne ansetzbar ist und der Drehmittelpunkt (6, 16) in einer Ebene liegt, die parallel zu und in einem Abstand von mehr als 11 mm vom Mittelbereich (8) der Stützfläche (3) des schädelseitigen Prothesenteils (1) angeordnet ist.

3. Prothese nach Anspruch 1 oder2, bei der der Drehmittelpunkt (6, 16) an einer Position relativ zur Stützfläche (3) des schädelseitigen Prothesenteils (1) derart angeordnet ist, daß, im implantierten Zustand, der Drehmittelpunkt (6, 16) zwischen 5 und 60 mm unterhalb der Mitte (26) des natürlichen Kiefergelenkkopfs liegt.

4. Prothese nach einem der vorhergehenden Ansprüche, bei der die Stützfläche (3) an Knochengewebe (4) der Gelenkpfanne ansetzbar ist und der Drehmittelpunkt (6, 16) in einer Ebene liegt, die parallel zu und in einem Abstand zwischen 13 und 68 mm vom Mittelbereich (8) der Stützfläche (3) des schädelseitigen Prothesenteils (1) angeordnet ist.

5. Prothese nach einem der vorhergehenden Ansprüche, bei der das kieferseitige Prothesenteil (2) um einen einzigen Drehmittelpunkt (6) verschwenkbar ist, der sich derart in einer zur Stützfläche (3) des schädelseitigen Prothesenteils (1) im wesentlichen festen Position befindet, daß, im implantierten Zustand, der Drehmittelpunkt (6) zwischen 5 und 25 mm, und vorzugsweise ungefähr 15 mm, unterhalb der Mitte (26) des natürlichen Kiefergelenkkopfs liegt.

6. Prothese nach einem der vorhergehenden Ansprüche, bei der das kieterseitige Prothesenteil (2) um einen einzigen Drehmittelpunkt (6) verschwenkbar ist, der sich in einer zur Stützfläche (3) des schädelseitigen Prothesenteils (1) im wesentlichen festen Position befindet, die Stützfläche (3) an Knochengewebe (4) der Gelenkpfanne ansetzbar ist und der Drehmittelpunkt (6, 16) in einer Ebene liegt, die parallel zu und in einem Abstand zwischen 13 und 33 mm, vorzugsweise ungefähr 23 mm, vom Mittelbereich (8) der Stützfläche (3) des schädelseitigen Prothesenteils (1) angeordnet ist.

7. Prothese nach einem der vorhergehenden Ansprüche, bei der eines der Prothesenteile (1, 2) einen kugelförmigen Hohlraum (9) aufweist und das andere der Prothesenteile ein kugelförmiges konvexes Element (10) aufweist, das gleitend bewegbar in den Hohlraum einsetzbar ist.

8. Prothese nach einem der Ansprüche 1-4, bei der das kieferseitige Prothesenteil (2) relativ zum schädelseitigen Prothesenteil (1) derart bewegbar ist, daß beim Drehen des kieferseitigen Prothesenteils (2) ein Momentan-Drehmittelpunkt des Kiefers sich entlang einer Polbahn (16) bewegt.

9. Prothese nach Anspruch 8, bei der die Polbahn (16) U-förmig ist, wobei das offene Ende der U-förmigen Polbahn (16) dem schädelseitigen Prothesenteil (1) zugewandt ist.

10. Prothese nach Anspruch 9, bei der zwei Stäbe (18, 19) gelenkig zwischen den Prothesenteilen (1, 2) angeordnet sind, wobei die Prothesenteile (1, 2) und die Stäbe (18, 19) ein Vierstab-System bilden.

11. Prothese nach Anspruch 8, bei der das kieferseitige Prothesenteil (2) ein Element (20) mit einer konvexen Lagerfläche aufweist und das schädelseitige Prothesenteil (1) mit einer sich hauptsächlich in einer Richtung erstreckenden Lagerfläche (21) versehen ist, wobei die Lagerflächen (21) derart angeordnet sind, daß, im implantierten Zustand, das Element (20) ventral und dorsal entlang der Lagerfläche (21) des schädelseitigen Prothesenteils (1) gleiten kann, und ein künstliche Band (22) an dem Element an einer von dessen Lagerfläche (21) beabstandeten Position angebracht ist.

12. Prothese nach Anspruch 8, bei der das kieferseitige Prothesenteil (2) ein Element (23) mit einer konvexen Lagerfläche aufweist, das schädelseitige Prothesenteil (1) mit einer sich hauptsächlich in einer Richtung erstreckenden Lagerfläche (21) und einen Anschlag (25) versehen ist, der von der Lagerfläche (24) beabstandet und vor dieser angeordnet ist, um von der Rückseite her einen benachbarten Teil des kieferseitigen Prothesenteils (2) zu stützen, wobei die Lagerfläche (24) derart angeordnet ist, daß, im implantierten Zustand, das Element (23) ventral und dorsal entlang der Lagerfläche (21) des schädelseitigen Prothesenteils (1) gleiten kann.

13. Prothese nach einem der vorhergehenden Ansprüche, bei der die Prothesenteile (1, 2) relativ zueinander quergerichtet in Richtungen verschiebbar sind, die im wesentlichen medial und lateral ausgerichtet sind, wenn die Prothese implantiert ist.

14. Prothese nach Anspruch 13, bei der eines der Prothesenteile (1, 2) einen kugelförmigen Hohlraum (9) aufweist und das andere der Prothesenteile ein kugelförmiges konvexes Element (10) aufweist, das gleitend bewegbar in den Hohlraum einsetzbar ist, wobei das gelenkpfannenseitige Prothesenteil (1) ein entlang einer Führung in der quergerichteten Verschiebungsrichtung (11) bewegbares Schiebeelement (12) aufweist, das mit dem kugelförmigen Hohlraum (9) oder dem kugelförmigen konvexen Element (19) versehen ist.

15. Kiefergelenkpfannenprothese zur Verwendung als Prothesenteil nach Anspruch 7, mit einer Stützfläche (3), die im Bereich der und/oder nahe der natürlichen Gelenkpfanne und/oder des Gelenkvorsprungs an Knochengewebe (4) gesetzt werden kann, einem kugelförmigen Hohlraum (9) oder konvexen Element (10), dessen Mittelpunkt (6), der durch den kugelförmigen Hohlraum (9) oder das konvexe Element (10) definiert ist, derart in einer relativ zur Stützfläche (3) im wesentlichen festen Position angeordnet ist, daß, im implantierten Zustand, der Drehmittelpunkt (6) zwischen 5 und 25 mm, und vorzugsweise ungefähr 15 mm, unterhalb der Mitte (26) des natürlichen Kiefergelenkkopfs liegt.

16. Kiefergelenkpfannenprothese nach Anspruch 15, bei der die Stützfläche (3) an Knochengewebe (4) der natürlichen Gelenkpfanne ansetzbar ist und der durch den kugelförmigen Hohlraum (9) oder das konvexe Element (10) definierte Mittelpunkt (6, 16) in einer Ebene liegt, die parallel zu und in einem Abstand zwischen 13 und 33 mm, vorzugsweise ungefähr 23 mm, vom Mittelbereich der Stützfläche (3) angeordnet ist.

## Revendications

1. Prothèse d'articulation temporo-mandibulaire comportant une partie de prothèse crânienne (1) comportant une surface de support (3) prévue pour reposer contre des tissus d'os (4) dans la zone de et/ou de façon adjacente à la fosse glénoïdale naturelle et/ou l'éminence articulaire et une partie de prothèse mandibulaire (2) prévue pour pouvoir pivoter par rapport à la partie de prothèse crânienne (1) dans une condition implantée, **caractérisée en ce que** la partie de prothèse mandibulaire (2) peut pivoter autour d'un centre de rotation (6, 16) qui se trouve dans une position par rapport à la surface de support (3) de la partie de prothèse crânienne (1) telle que, dans une condition implantée, ledit centre de rotation (6) est disposé plus bas que le centre (26) du condyle mandibulaire naturel.

2. Prothèse selon la revendication 1, dans laquelle la surface de support (3) est prévue pour être ajustée sur les tissus d'os (4) de la fosse et le centre de rotation (6, 16) est disposé dans un plan parallèle à et à une distance supérieure à 11 mm d'une partie centrale (8) de ladite surface de support (3) de la partie de prothèse crânienne (1).

3. Prothèse selon la revendication 1 ou 2, dans laquelle ledit centre de rotation (6, 16) est disposé dans une position par rapport à la surface de support (3) de la partie de prothèse crânienne (1) telle que, dans une condition implantée, ledit centre de rotation (6, 16) se trouve entre 5 et 60 mm plus bas que le centre (26) du condyle mandibulaire naturel.

4. Prothèse selon l'une des revendications précédentes, dans laquelle la surface de support (3) est prévue pour être montée sur les tissus d'os (4) de la fosse et le centre de rotation (6, 16) est disposé dans un plan parallèle à et à une distance entre 13 et 68 mm d'une partie centrale (8) de la surface de support (3) de la partie de prothèse crânienne (1).

5. Prothèse selon l'une des revendications précédentes, dans laquelle la partie de prothèse mandibulaire (2) peut pivoter autour d'un unique centre de rotation (6) qui est disposé dans une position sensiblement fixe par rapport à la surface de support (3) de la partie de prothèse crânienne (1) telle que, dans une condition implantée, ledit centre de rotation (6) est disposé entre 5 et 25 mm, et de préférence environ 15 mm, plus bas que le centre (26) du condyle mandibulaire naturel.

6. Prothèse selon l'une des revendications précédentes, dans laquelle la partie de prothèse mandibulaire (2) peut pivoter autour d'un unique centre de rotation (6) qui est disposé dans une position sensiblement fixe par rapport à la surface de support (3) de la partie de prothèse crânienne (1), la surface de support (3) est prévue pour être montée sur les tissus d'os (4) de la fosse et le centre de rotation (6, 16) est disposé dans un plan parallèle à et à une distance entre 13 et 33 mm, et de préférence environ 23 mm, d'une partie centrale (8) de la surface de support (3) de la partie de prothèse crânienne (1).

7. Prothèse selon l'une des revendications précédentes, dans laquelle une desdites parties de prothèse (1, 2) comporte une cavité en forme de sphère (9) et l'autre desdites parties de prothèse comporte un élément convexe en forme de sphère (10) prévu pour être logé dans ladite cavité avec un ajustement coulissant.

8. Prothèse selon l'une des revendications 1 à 4, dans laquelle la partie de prothèse mandibulaire (2) est mobile par rapport à la partie de prothèse crânienne (1) de telle sorte que, lors de la rotation de la partie de prothèse mandibulaire (2), un centre de rotation instantané de la mandibule se déplace le long d'une base roulante (16).

9. Prothèse selon la revendication 8, dans laquelle la base roulante (16) est en forme de U, l'extrémité ouverte de la base roulante en forme de U (16) étant orientée vers la partie de prothèse crânienne (1).

10. Prothèse selon la revendication 9, dans laquelle deux barres (18, 19) sont articulées entre lesdites parties de prothèse (1, 2), les parties de prothèse (1, 2) et les barres (18, 19) formant un système à quatre barres.

11. Prothèse selon la revendication 8, dans lequel la partie de prothèse mandibulaire (2) comprend un élément (20) avec une surface de palier concave, la partie de prothèse crânienne (1) est pourvue d'une surface de palier (21) qui s'étend principalement dans une direction, les surfaces de palier (21) étant disposées de telle sorte que, dans une condition implantée, l'élément (20) peut coulisser de manière ventrale et dorsale le long de la surface de palier (21) de la partie de prothèse crânienne (1), et un ligament artificiel (22) est fixé sur l'élément dans une position espacée de sa surface de palier (21).

12. Prothèse selon la revendication 8, dans laquelle la partie de prothèse mandibulaire (2) comporte un élément (23) avec une surface de palier convexe, la partie de prothèse crânienne (1) est pourvue d'une surface de palier (24) s'étendant principalement dans une direction et une retenue (25) espacée de et à l'avant de sa surface de palier (24) afin de supporter de manière postérieure une partie adjacente de la partie de prothèse mandibulaire (2), la surface de palier (24) étant disposée de telle sorte que, dans une condition implantée, l'élément (23) peut coulisser de manière ventrale et dorsale le long de la surface de palier (24) de la partie de prothèse crânienne (1).

13. Prothèse selon l'une des revendications précédentes, dans laquelle les parties de prothèse (1, 2) sont prévues pour un déplacement en translation l'une par rapport à l'autre dans des directions (11) qui sont essentiellement orientées de manière médiane et latérale lorsque la prothèse est dans une condition implantée.

14. Prothèse selon la revendication 13, dans laquelle une desdites parties de prothèse (1, 2) comporte une cavité en forme de sphère (9) et l'autre desdites parties de prothèse comporte un élément convexe en forme de sphère (10) prévu pour être logé dans ladite cavité (9) avec un ajustement coulissant, et la partie de prothèse de fosse glénoïdale (1) comporte un élément de déplacement (12) mobile le long d'un guide (13) dans ladite direction (11) du déplacement en translation et pourvu de la cavité (9) ou de l'élément convexe (10) en forme de sphère.

15. Prothèse de fosse glénoïdale pour utilisation comme partie d'une prothèse selon la revendication 7, comportant une surface de support (3) prévue pour être logée contre les tissus d'os (4) dans la zone de ou de façon adjacente à la fosse glénoïdale naturelle et/ou l'éminence articulaire, une cavité (9) ou un élément convexe (10) en forme de sphère, le centre de rotation (6) défini par ladite cavité (9) ou ledit élément convexe (10) en forme de sphère étant disposé dans une position sensiblement fixe par rapport à la surface de support (3) telle que, dans une condition implantée, ledit centre est disposé entre 5 et 25 mm, et de préférence environ 15 mm, plus bas que le centre (26) du condyle mandibulaire naturel.

16. Prothèse de fosse glénoïdale selon la revendication 15, dans laquelle ladite surface de support (3) est prévue pour être montée sur des tissus d'os (4) de la fosse glénoïdale naturelle, et dans laquelle le centre (6) défini par ladite cavité (9) ou ledit élément convexe (10) en forme de sphère est disposé dans un plan parallèle à et à une distance entre 13 et 33 mm, et de préférence environ 23 mm, d'une partie centrale de la surface de support (3).
